(19) Europäisches Patentamt
European Patent Office
Office européen des brevets

(11) **EP 4 230 745 A1**

(12) **EUROPEAN PATENT APPLICATION**
published in accordance with Art. 153(4) EPC

(43) Date of publication:
**23.08.2023 Bulletin 2023/34**

(21) Application number: **21880127.2**

(22) Date of filing: **13.10.2021**

(51) International Patent Classification (IPC):
*C12Q 1/68* [(2018.01)]    *G16H 50/70* [(2018.01)]
*G01N 33/493* [(2006.01)]    *G01N 33/50* [(2006.01)]
*G16Y 20/20* [(2020.01)]    *G16Y 40/20* [(2020.01)]

(52) Cooperative Patent Classification (CPC):
**C12Q 1/68; G01N 33/493; G01N 33/50;
G16H 50/70; G16Y 20/20; G16Y 40/20**

(86) International application number:
**PCT/JP2021/037799**

(87) International publication number:
**WO 2022/080389 (21.04.2022 Gazette 2022/16)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **14.10.2020 JP 2020172934**

(71) Applicant: **Craif Inc.**
**Tokyo 1130034 (JP)**

(72) Inventors:
• **ICHIKAWA, Yuki**
  **Tokyo 113-0033 (JP)**
• **YAMAGUCHI, Hiroki**
  **Tokyo 113-0033 (JP)**
• **MIZUNUMA, Mika**
  **Tokyo 113-0033 (JP)**

(74) Representative: **Michalski Hüttermann & Partner
Patentanwälte mbB
Kaistraße 16A
40221 Düsseldorf (DE)**

(54) **METHOD FOR EVALUATING LIKELIHOOD OF OBSERVATION VALUE AND PROGRAM**

(57)    The present disclosure provides a method for evaluating a likelihood that a subject belongs to a group for a classification attribute having a binary classification. The method includes: receiving a subject score for an observation value of the subject; acquiring sensitivity and specificity of the subject score with the subject score as a parameter, by using a relational expression established between the sensitivity and the specificity with a score for the observation value as a parameter; acquiring a prior probability of an attribute of the subject; and acquiring a likelihood of belonging to a classification attribute specific to the subject based on the sensitivity, the specificity, and the prior probability of the subject.

EP 4 230 745 A1

FIG. 1

**Description**

Technical Field

The present disclosure relates to a method, a program, and a system for evaluating certainty of an observation value.

Background Art

Examination results have been compared with an ROC curve to determine whether a subject is positive or negative. A specific point on the ROC curve (point closest to the left corner, point defined by Youden Index) has been used as a cutoff point. It has been common to return a positive result if an observation value is higher than the cutoff point and a negative result if the observation value is lower than the cutoff point. Based on that cutoff point, a positive predictive value (PPV) has been calculated. However, this positive predictive value is a value unique to the ROC curve, and is merely a value for evaluating an evaluation system.

Summary of Invention

A method is desired for more appropriately evaluating whether a subject is positive or negative based on an examination result, that is, the result of the subject.
According to an embodiment of the present disclosure, there is provided a novel method for evaluating a likelihood (certainty) that a subject belongs to a group. In some embodiments, the method may include receiving a subject score for an observation value of the subject. In some embodiments, the method may include acquiring sensitivity and specificity of the subject score with the subject score as a parameter, by using a relational expression established between the sensitivity and the specificity with a score for the observation value as a parameter. In some embodiments, the method may include acquiring a prior probability of an attribute of the subject. In some embodiments, the method may include acquiring a likelihood of belonging to a classification attribute specific to the subject based on the sensitivity, the specificity, and the prior probability of the subject.
Additional aspects and advantages of the present disclosure will become readily apparent to those skilled in this art from the following detailed description, wherein only illustrative embodiments of the present disclosure are illustrated and described. As will be realized, the present disclosure is capable of other and different embodiments, and its several details are capable of modifications in various obvious respects, all without departing from the present disclosure. Accordingly, the drawings and description are to be regarded as illustrative in nature and not as restrictive.

Brief Description of Drawings

[Fig. 1] Fig. 1 is a flowchart illustrating an evaluation method according to an embodiment of the present disclosure.
[Fig. 2] Fig. 2 is a block diagram illustrating a computer control system according to an embodiment of the present disclosure.

Description of Embodiments

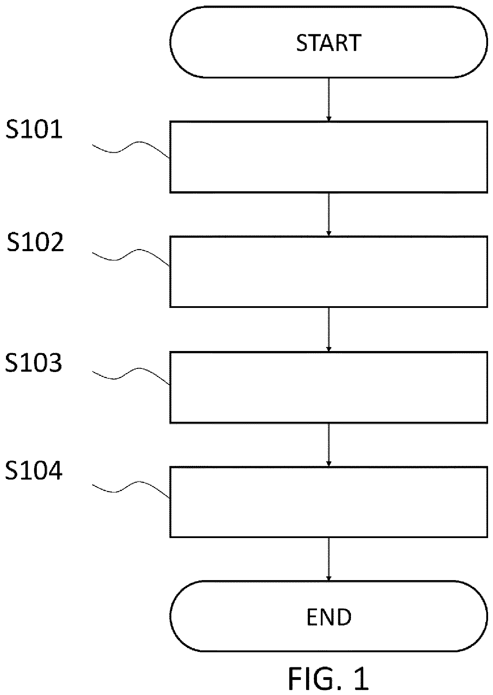

Fig. 1 illustrates a flowchart of a novel method for evaluating a likelihood (certainty) that a subject belongs to a group according to an embodiment of the present disclosure. In step S101, a subject score for an observation value of the subject is received. In step S102, sensitivity and specificity of the subject score with the subject score as a parameter is acquired , by using a relational expression established between the sensitivity and the specificity with a score for the observation value as a parameter. In step S103, a prior probability of an attribute of the subject is acquired. In step S104, a likelihood of belonging to a classification attribute specific to the subject is acquired based on the sensitivity, the specificity, and the prior probability of the subject.

<Observation>

"Observation" used in the present disclosure is not limited to "observing" in a narrow sense, and generally refers to observation, measurement, analysis, and the like in biology, medicine, pharmacy, biochemistry, physics, chemistry, electrical engineering, and optics.

<Examination>

In some embodiments, the observation may be a clinical examination.

The clinical examination includes sample examination, biological examination, image diagnosis, pathological diagnosis, physical examination, psychological examination, and other examination for obtaining relevant information with or without the purpose of determining the presence or absence of a disease or diagnosis.

The sample examination includes biochemical examination, hematological examination, urine/stool examination, immunological examination, microbiological examination, and the like.

In the present disclosure, a body fluid used for the examination means a body fluid obtained from the subject or a sample derived from the body fluid. The body fluid may be, but not limited to, blood, serum, plasma, lymph, tissue fluid such as inter-tissue fluid, intercellular fluid, interstitial fluid, body cavity fluid, serous cavity fluid, pleural fluid, peritoneal fluid, pericardial fluid, cerebrospinal fluid (spinal fluid), joint fluid (synovial fluid), hydatoid (aqueous humor). The body fluid may be a digestive fluid such as saliva, gastric juice, bile, pancreatic juice, intestinal fluid, and may be sweat, tears, nasal discharge, urine, semen, vaginal fluid, amniotic fluid, or milk. The body fluid may be an animal body fluid or a human body fluid.

The biological examination includes respiratory circulatory function examination, ultrasonography, various examinations using a monitoring device, electroencephalography, neuromuscular examination, otorhinolaryngological examination, ophthalmological examination, dermatological examination, clinical psychological/neuropsychological examination, stress examination, examination using radioisotopes, endoscopic examination, and the like. In some embodiments, the biological examination may be a liquid biopsy.

In some embodiments, the observation value may be an amount, frequency, or other examination value related to expression of a gene in a genetic examination.

The gene may be a nucleic acid (at least one of DNA and RNA).

The RNA may be messenger RNA (mRNA), transfer RNA (tRNA), ribosomal RNA (rRNA), microRNA (miRNA), or the like.

In the genetic examination, a subject's body fluid (e.g., blood, saliva, or urine) may be acquired, and the amount (relative amount, absolute amount) of a predetermined or given nucleic acid may be measured. The nucleic acid may be amplified. The nucleic acid may be measured by using genetic analyzer such as a DNA chip (also referred to as a microarray) or a sequencer.

The genetic examination may include genetic mutation examination. A copy number change may be measured as a genetic mutation. A number/expression level change of single nucleotide polymorphisms (SNPs) may be measured. A fusion gene may also be measured. For example, it may be determined whether a fusion has occurred at a predetermined gene or base site. The number of fusion genes may be measured. A chromosomal abnormality may be measured. The presence or absence of a chromosomal abnormality, its amount, frequency, or the like within a predetermined region may be measured. A chromosomal abnormality may be a change in structure or a change in chromosome number, or both. A tumor mutation burden (TMB) may be measured. The number of oncogenes or a TMG score may be measured. The amount of an epigenetic change, such as methylation (number of sites, frequency at a predetermined site), acetylation, or the like may be measured. The number of sites at which these mutations have occurred or the amount of change at a predetermined site may be measured. Analysis or examination for microsatellite instability (MSI) may be performed. The number or frequency of altered bases in a microsatellite region may be measured. A splicing abnormality may be measured. The presence or absence of the abnormality may be measured, and the number (number or number of bases), absolute number, frequency, or the like of the sites may be measured.

If the observation is evaluated by a numerical value, the score may be the observation value or a value converted from the observation value. If the observation does not give a numerical value, the score may be obtained by digitizing an observation result by an evaluation method.

The score may be a value of the examination result itself or a processed value. The score may be a normalized value. The score may be calculated based on a value or evaluation of the examination result. The score may be calculated based on results of a plurality of examinations. In some embodiments, they may be combined to calculate a score using software such as machine learning. The score may be continuous or noncontinuous (discrete numbers, e.g. binary 0/1). In some embodiments, the amount of a predetermined gene (e.g., RNA) in a body fluid in the genetic examination may be used as a score, or a score determined from a gene expression level profile may be used.

In some embodiments, a method of the present disclosure may include providing a binary classification based on a score for the observation value. In some embodiments, the method of the present disclosure may include providing a multiclass classification based on a score for the observation value.

The binary (or multiclass) classification may be prepared or provided in advance or independently of the observation of the subject. A predetermined classification may be obtained for the score of the observation value.

<Relational Expression>

In some embodiments, a relational expression with the score as a parameter may be used.

The parameter may also be referred to as a threshold or cutoff point.

In some embodiments, a relational expression between a value for one from the binary classification and a value for the other may be used. In some embodiments, a relational expression between either one of TPR (true positive rate) and FPR (false positive rate) and either one of FPR (false positive rate) or TNR (true positive rate) in the binary classification may be used. For example, any one of a relational expression between TPR and FPR, a relational expression between TPR and TNR, a relational expression between FNR and FPR, and a relational expression between FNR and TNR may be used. For example, a relational expression between sensitivity (TPR) and specificity (1 - FPR) may be used. For example, a relational expression between sensitivity (TPR) and (1 - specificity) = FPR may be used.

In some embodiments, the relational expression may be represented by an ROC (Receiver Operating Characteristic) or an ROC curve.

One feature of the present disclosure may be to use the sensitivity, the specificity, or the like with the score (subject score) based on the observation value of the subject as a score of the relational expression (that is, subject score = score of relational expression). Accordingly, for example, even if an expression used in Bayesian statistics (Bayesian estimation, Bayesian probability) is used, an evaluation value obtained therefrom is not a value for evaluating a statistical system, but the observation value of the subject can be used as an evaluation.

<Prior Probability>

The prior probability in Bayesian statistics may be investigated prior to (a priori) the observation of the subject. In some embodiments, the prior probability may be acquired after the observation of the subject. That is, it may be used as the prior probability. In some embodiments, the prior probability may be acquired after the observation of the subject, for example, objectively. The prior probability obtained after the observation may be used to calculate the likelihood, or a new prior probability may be used to recalculate a previously obtained observation result.

In some embodiments, the likelihood that the observation value is positive may be expressed as a conditional probability of a positive predictive value.

<Likelihood>

In some embodiments, the probability (likelihood) that the subject actually belongs to a group (class) may be calculated using Bayesian statistics. The likelihood may be calculated using a conditional probability calculation formula in Bayesian statistics, including the prior probability.

For example, in order to evaluate whether a subject has a disease by a clinical examination, a calculation formula can be expressed as follows:

[EQ. 1]

$$P(\text{with disease}|\text{positive})$$
$$= \frac{P(\text{positive}|\text{with disease}) \times P(\text{with disease})}{P(\text{positive})} \quad \text{Eq. 1}$$

Therefore, the above equation can be rewritten as follows:

[EQ. 2]

$$PPV = \frac{Se \times \alpha}{Se \times \alpha + (1 - Sp) \times (1 - \alpha)} \quad \text{Eq. 2}$$

In the above equation, Se, Sp, and $\alpha$ represent sensitivity, specificity, and prevalence, respectively.

Similarly, a negative predictive value can be expressed as follows:

[EQ. 3]

P(without disease|negative)

$$= \frac{P(negative|without\ disease)\ \times\ P(without\ disease)}{P(negative)} \qquad Eq.\ 3$$

Therefore, the above equation can be rewritten as follows:
[EQ. 4]

$$NPV = \frac{Sp \times (1-\alpha)}{(1-Se) \times \alpha + Sp \times (1-\alpha)} \qquad Eq.\ 4$$

However, the sensitivity and the specificity illustrated in the present disclosure are not values unique to the ROC curve (e.g., point closest to the left corner on the ROC curve, Youden index, or the like). They are the sensitivity (subject sensitivity) and the specificity (subject specificity) calculated from a value on the ROC curve corresponding to the score of the observation value of the subject. Therefore, being different from those in the classical meaning, the above equations can be called modP (with disease|positive), modPPV, modN (without disease|negative), and modNPV, respectively. The names are not limited to these, and other names may be used.

In some embodiments, the likelihood may be represented by one function or may be represented by a plurality of functions. A total of a plurality of functions may be referred to as one function. For example, a plurality of functions may be used in combination. For example, a plurality of functions may be defined by a range of scores. For example, the PPV and the NPV may be adapted according to the range of scores. For example, the PPV may be used for scores above a certain value, and the NPV may be used for scores below that value. In some embodiments, a likelihood ratio may be used, such as a positive likelihood ratio, a negative likelihood ratio, or the like.

<Multiclass Classification>

According to some embodiments of the present disclosure, the likelihood that the subject belongs to each class defined by the multiclass classification may also be evaluated. In some embodiments, a one-versus-one model may be used. In some embodiments, a one-versus-rest model may be used.

The probability that a class i is positive in an N-class classification using the one-versus-rest model can be expressed as follows:
[EQ. 5]

P(belonging to class $i$|class $i$ positive)

$$= \frac{P(class\ i\ positive|belonging\ to\ class\ i)\ \times\ P(belonging\ to\ class\ i)}{P(class\ i\ positive)} \qquad Eq.\ 5$$

Then, a value (modPPV) obtained by substituting the score of the subject into this may be adopted as the likelihood.
[EQ. 6]

$$modPPV(belonging\ to\ class\ i|class\ i\ positive) \qquad Eq.\ 6$$

Table 1 illustrates a cross-tabulation table for N = 3.

[Table 1]

Table 1

| | | Condition | | |
|---|---|---|---|---|
| | | Class 1 | Class 2 | Class 3 |
| Test (Observed) | Class 1 | True Class 1 | False Class 1 (2) | False Class 1(3) |
| | Class 2 | False Class 2 (1) | True Class 2 | False Class 2(3) |
| | Class 3 | False Class 3 (1) | False Class 3 (2) | True Class 3 |

For example, the likelihood that "class 1" is true (i.e., "true class 1") based on a test result (true class 1 predictive value) can be expressed based on the one-versus-rest model and Bayesian statistics as follows:
[EQ. 7]

$$\text{True class 1 predictive value}$$
$$= \frac{\text{P(true class 1)} \times \alpha 1}{\text{P(true class 1)} \times \alpha 1 + [\text{P(false class 1(2)} + \text{P(false class 1(3))}] \times (1 - \alpha 1)} \quad \text{Eq. 7}$$

Similarly, the likelihood that "class 1" is false (i.e., "false class 1") based on a test result (false class 1 predictive value) can be expressed as follows:
[EQ. 8]

$$\text{False class 1 predictive value}$$
$$= \frac{[\text{P(false class 1(2))} + \text{P(false class 1(3))}] \times (1 - \alpha 1)}{\text{P(true class 1)} \times \alpha 1 + [\text{P(false class 1(2))} + \text{P(false class 1(3))}] \times (1 - \alpha 1)} \quad \text{Eq. 8}$$

Extending the above, in a general case of an N-class classification (N classes), the likelihood that the class i is true based on a test result (true class i predictive value) can be expressed as follows: [EQ. 9]

$$\text{True class } i \text{ predictive value}$$
$$= \frac{\text{P(true class } i) \times \alpha i}{\text{P(true class } i) \times \alpha i + \sum_{j \neq i}^{N} [\text{P(false class } i(j) \times (1 - \alpha j)]} \quad \text{Eq. 9}$$

Similarly, the likelihood that "class i" is false (i.e., "false class i") based on a test result (false class i predictive value) can be expressed as follows: [EQ. 10]

$$\text{False class } i \text{ predictive value}$$
$$= \frac{\sum_{j \neq i}^{N} [\text{P(false class } i(j) \times (1 - \alpha j)]}{\text{P(true class } i) \times \alpha i + \sum_{j \neq i}^{N} [\text{P(false class } i(j) \times (1 - \alpha j)]} \quad \text{Eq. 10}$$

<Examples>

With an embodiment of the present disclosure, it was evaluated whether a subject has cancer based on expression of RNA. The results will be described below.

[Table 2]

Table 2

| | | | | Method of the present example | | | Conventional method | | |
|---|---|---|---|---|---|---|---|---|---|
| Subject | Attribute | Prevalence | Score | Sensitivity | Specificity | **ModPPV** | Sensitivity | Specificity | **Conventional PPV** |
| A | Male in their 20s | 0.00055% | **0.62** | 9.38% | 99.99% | **0.3808%** | 87.75% | 87.75% | **0.0039%** |
| B | Male in their 20s | 0.00055% | **0.02** | 86.05% | 89.30% | **0.0044%** | 87.75% | 87.75% | **0.0039%** |
| C | Male in their 50s | 0.06260% | **0.54** | 15.93% | 99.96% | **18.2769%** | 87.75% | 87.75% | **0.4466%** |
| D | Male in their 50s | 0.06260% | **0.12** | 75.25% | 94.98% | **0.9295%** | 87.75% | 87.75% | **0.4466%** |

Table 2 illustrates scores obtained by a certain lung cancer biomarker examination method and their evaluation values for subjects (A to D). The ROC curve between the lung cancer examination results and the examination results (scores) was obtained in advance.

The subjects A and B have the same attribute of male in their 20s. The subjects C and D have the same attribute of male in their 50s. Prevalence is defined by attributes of the subject. The prevalence among men in their 20s is 0.00055 %. The prevalence among men in their 50s is 0.06260 %.

The examination results showed that among men in their 20s, the subject A had a relatively high score value (0.62), and the subject B had a relatively low score value (0.02). The examination results showed that among men in their 50s, the subject C had a relatively high score value (0.54), and the subject D had a relatively low score value (0.12).

Here, the score was calculated to indicate "0" if it corresponds to the "threshold" or "cutoff point" separating positive and negative results. Thus, a score close to 0 means a value close to the threshold (the subjects B and D). On the other hand, a score far from 0 means a value far from the threshold value. Alternatively, a positive score far from 0 suggests a higher probability of being positive (the subjects A and C).

The sensitivity and the specificity based on the Youden index are shown in the ROC curve of the lung cancer examination results. The sensitivity and the specificity depend on the same index. In Table 2, the sensitivity and the specificity corresponding to the Youden index happened to be the same. However, in general, the sensitivity and the specificity may have different values.

The conventional positive predictive value (PPV) is calculated based on the sensitivity and the specificity based on the Youden index and the prevalence. As a result, the subjects A and B belonging to the same attribute, male in their 20s, have the same prevalence and therefore have the same value of PPV. In other words, the subjects A and B have the same value of PPV (0.0039 %) even though they have different scores. Similarly, the subjects C and D belonging to the same attribute, male in their 50s, have the same prevalence. Therefore, they have the same value of PPV (0.4466 %) even though they have different scores.

Using the conventional PPV, only if the score is greater than the threshold, both (the subjects A and B, male in their 20s, or the subjects C and D, male in their 50s) are determined positive. This result does not depend on the magnitude of the score as the examination result. Furthermore, if a conventional PPV calculation formula is used at that time, the same value of PPV is given in both cases. This is because the PPV is a parameter representing characteristics of the ROC curve. The conventional method was unable to represent differences in examination scores. The conventional method was unable to individually express the likelihood that they have a disease.

On the other hand, the evaluation value according to an embodiment of the present disclosure uses the sensitivity and the specificity with the score as a parameter of the ROC curve. The subjects A to D have different scores and therefore differ from each other in the sensitivity and the specificity (Table 2). The evaluation value was obtained by substituting the sensitivity and the specificity and the prevalence into a general PPV calculation formula. Thus, it is different from the conventional PPV.

As illustrated in Table 2, the subjects A to D were given different evaluation values (0.3808 %, 0.0044 %, 18.2769 %, and 0.9295 %, respectively). The subjects A and B having the same attribute have different evaluation values. Similarly, the subjects C and D having the same attribute have different evaluation values. In this way, for a plurality of subjects having the same attribute but different scores, it is possible to give an evaluation value that reflects the level of the score and the prevalence rate and is the likelihood of having a disease. In addition, the subject D obtained a higher score, but a lower evaluation value than the subject A. In this way, comparable evaluation values (for example, the likelihood of having a disease) can be given to a plurality of subjects belonging to different attributes.

Thus, it is shown that the method of the present disclosure can be used to more appropriately evaluate the likelihood that a subject has cancer than the conventional method.

<Other Applications>

The method and the like of the present disclosure are applicable to a clinical examination, and in addition, other evaluations.

In some embodiments, the observation may be image diagnosis. Some embodiments of the present disclosure may also be applied to medical image processing (image diagnosis, image-based disease diagnosis).

For example, the image includes, but is not limited to, an optical image, an ultrasound image, an X-ray image, a magnetic resonance image (MRI), and a radioisotopic (RI) image. The medical image diagnosis may be general radiography (commonly referred to as X-ray examination). For example, it may be a simple X-ray imaging diagnosis or a dental panoramic X-ray imaging diagnosis. Other medical image diagnosis may be mammography, computerized tomography (CT), gastrointestinal contrast examination (barium meal examination), interventional radiology (IVR), or the like.

The score in image diagnosis may be determined based on a continuous certainty factor method. The score may be determined based on a computational technique or an algorithm such as artificial intelligence or software.

Some embodiments of the present disclosure may be applied to various evaluations other than the clinical examination or similar examination.

Some embodiments of the present disclosure provide an application to personal authentication. In some embodiments, observation for biometric authentication may be made.

For example, the biometric authentication includes, but is not limited to, fingerprint authentication, knuckle pattern authentication, vein authentication (with finger, palm, back of hand, or the like) authentication, palm (physical features of hand) authentication, iris authentication, two dimensional/three dimensional face authentication, optical or X-ray dental image authentication, voice authentication, handwriting authentication, and the like.

An image, a sound waveform, and other data may be used as the observation value.

Some embodiments of the present disclosure provide applications to meteorological forecasting. In some embodiments, the likelihood that certain weather will occur may be evaluated. For example, the probability of precipitation, the amount of solar radiation, the wind speed, or the like may be predicted based on various types of weather data (pressure pattern, humidity, temperature, wind speed, wind direction, state of jet airflow, sea temperature, tidal current, topography, or the like).

Some embodiments of the present disclosure provide applications to natural disaster prediction. In some embodiments, the likelihood that a natural disaster will occur may be evaluated. For example, the likelihood of a natural disaster such as heavy rain, flood, landslide, forest fire, earthquake, and eruption may be evaluated based on various geological data, weather data, radiation data, planetary data, or the like.

Some embodiments of the present disclosure provide applications to prediction of occurrence of a problem in a production line in industrial fields such as mechanical, electrical, chemical, and pharmaceutical fields. In some embodiments, the likelihood that a certain problem will occur may be evaluated. For example, the likelihood of occurrence of a problem in industrial production such as a machine tool, an electrical system, or a chemical reaction may be evaluated based on various operation data or abnormal signals (e.g., machine vibration, sound, current, temperature, characteristics of a product, or other state change of a device) .

Some embodiments of the present disclosure provide applications to variation prediction of a stock price, a national or regional growth rate, an inflation rate, and an interest rate. Some embodiments of the present disclosure provide an application to horse race result prediction. The present disclosure is not limited to the applications described herein. The present disclosure may have other applications.

<System>

The present disclosure also provides a computer control system that is programmed or otherwise configured to perform the method provided herein, such as a method for evaluating the likelihood (certainty) that a subject belongs to a group.

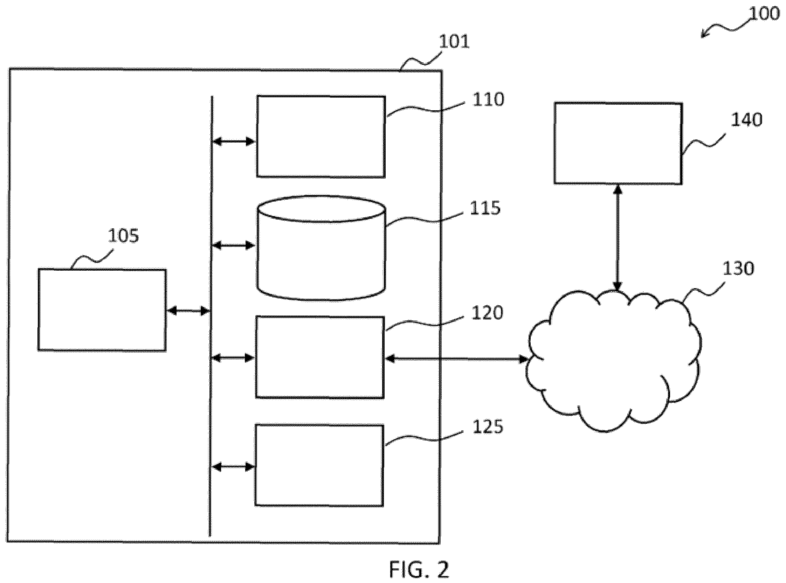

Fig. 2 illustrates an embodiment of a computer system 101 connected to a network 130 for performing the evaluation method of the present disclosure. The computer system 101 illustrated in Fig. 2 is communicatively connected to the network 130 via which communication with a user interface 140 is possible. The whole functions as a network system 100.

The computer system 101 includes a central processing unit (CPU, "processor" and "computer processor" herein) 105, a memory or memory location 110, an electronic storage unit 115, a communication interface 120 for communicating with one or more other systems, and a peripheral device 125.

The CPU 105 can be a single core or multi-core processor, or a plurality of processors for parallel processing. The CPU may be a GPU. For example, the memory 110 may be, but not limited to, random access memory, read-only memory, or flash memory. The storage unit 115 can be a data storage unit (or data repository) for storing data. For example, the storage unit 115 may be, but not limited to, a hard disk, a magnetic tape, or the like. For example, the communication interface 120 may be, but not limited to, a network adapter or the like. The communication interface 120 can communicate with the user interface 140 via the network 130. For example, the peripheral device may be, but not limited to, a cache, other memory, data storage, and/or an electronic display adapter.

A plurality of user interfaces 135 may be communicatively connected to the network 130. The user interface 135 may be located within or connected to the computer system 101.

In the computer system 101 in Fig. 1, the memory 110, the storage unit 115, the interface 120, and the peripheral device 125 are in communication with the CPU 105 via a communication bus (solid lines), such as a motherboard.

One or more components of the system 101 may communicate in other forms. One or more components of the system 101 may be substantially co-located and communicatively connected via, for example, the network 130.

The computer system 101 in Fig. 1 can be operatively coupled to the computer network ("network") 130 using the communication interface 120. The network 130 can be the Internet, an intranet and/or extranet, or an intranet and/or extranet in communication with the Internet. The network 130 in some cases is a telecommunication and/or data network. The network 130 may include one or more computer servers that may enable distributed computing, such as cloud computing. The network 130, in some cases with the aid of the computer system 101, can implement a peer-to-peer network that can enable a device coupled to the computer system 101 to operate as a client or a server.

The CPU 105 can execute a series of machine-readable instructions, which can be executed by a program or software. The instructions may be stored in a memory location, such as the memory 110. The instructions may be directed to the CPU 105, which may then be programmed or otherwise configured to implement the method of the present disclosure. Examples of operations performed by the CPU 105 may include fetch, decode, execute, and write-back.

The CPU 105 may be part of a circuit, such as an integrated circuit. One or more other components of the system 101 may be included in the circuit. In some cases, the circuit is an application specific integrated circuit (ASIC).

The storage unit 115 can store files such as a driver, a library, and a saved program. The storage unit 115 can store user data such as, for example, a user preference and a user program. In some cases, the computer system 101 may include one or more additional data storage units that are external to the computer system 101, such as one located on a remote server that is in communication with the computer system 101 via an intranet or the Internet. The computer system 101 can communicate with one or more remote computer systems via the network 130.

The method described herein can be implemented by way of machine- (e.g., computer processor) executable code stored on an electronic storage location of the computer system 101, such as, for example, the memory 110 or the electronic storage unit 115. Alternatively, the machine-readable code can be provided in the form of software. During use, the code can be executed by the processor 105. In some situations, the code may be retrieved from the storage device 115 and stored in the memory 110 for ready access by the processor 105. In some situations, the electronic storage device 115 can be excluded, and machine-executable instructions are stored in the memory 110.

The code may be pre-compiled and configured for use on a machine with a processor configured to execute the code, or may be compiled at runtime. The code can be provided in a programming language that can be selected to enable execution of the code in a pre-compiled or compiled form.

<Working of System>

Using the network system 100 including such a system 101, it is possible to evaluate the likelihood that a subject belongs to a certain group (class) based on an observation result of the subject.

First, the subject is observed (not illustrated). Subsequently, information related to observation, such as an observation value and information related to the subject, may be entered into the user interface 135 either passively by an operator or the like, or automatically or upon a command from another communicatively connected device by an operator or the like.

The entered information related to observation is entered at the user interface 140, conveyed to the computer system 101 via the network 130, and received by the communication interface 120. The information received by the communication interface 120 is temporarily stored in the memory 110.

In the embodiment illustrated in Fig. 2, data already obtained in relation to the observation is stored in the storage 115. For example, information about an attribute of the subject, information about the observation such as examination, a value or data obtained from the observation, a score for an observation value, statistical information such as raw data and a statistical value thereof, an algorithm of a classifier, statistical data after classification, data expressed by a binary or multiclass classification, information related to Bayesian statistics such as a parameter between classes such as a threshold, sensitivity, or specificity, a relational expression between them such as an ROC curve, or the like is stored in the storage 115.

The CPU 105 accesses the storage 115 on the basis of the received information on the attribute of the subject, and acquires a prior probability (prevalence in the case of disease examination) related to the attribute of the subject, information related to a multiclass classification such as an ROC curve, an evaluation formula of the likelihood, and the like. The acquired information is temporarily stored in the memory 110.

The CPU 105 uses the acquired relational expression, parameter, and information on the subject to calculate or evaluate the likelihood that the subject belongs to a certain group (class).

The CPU 105 may convey an evaluation result of the likelihood to the peripheral device 125 for display on a display device, or from the communication interface 120 via the network 130 to another device, such as the user interface 140.

The CPU 105 may store the evaluation result of the likelihood and the information about the subject in the storage 115. The data newly stored in the storage 115 may be incorporated into a population and used in the next evaluation.

The computer system 101 can be programmed or otherwise configured to adjust one or more parameters to evaluate the likelihood that the subject belongs to a group (class) based on the observation result of the subject.

Aspects of the system and the method provided herein, such as the computer system 101, can be embodied in programming. Various aspects of the technology may be thought of as "products" or "manufactures" typically in the form of a machine- (or processor) executable code and/or associated data that is carried on or embodied in a type of machine-readable medium. The machine-executable code can be stored on an electronic storage unit, such as memory (e.g., read-only memory, random-access memory, flash memory) or a hard disk. A "storage" type medium can include any or all of a tangible memory of a computer, processor, or the like, or an associated module thereof, such as various semiconductor memories, tape drives, disk drives, and the like, which can provide non-transitory storage at any time for software programming. All or part of the software may specifically be communicated over the Internet or various other communication networks. Such communications may, for example, enable loading of the software from a computer or processor into another computer, for example, from a management server or host computer into a computer platform of an application server. Thus, another type of medium that may bear software elements includes light, electricity, and electromagnetic waves, such as one used across physical interfaces between local devices, over wired and optical landline networks, and over various air links. Physical elements that carry such a wave, such as a wired or wireless link, an optical link, or the like, can also be considered as a medium that bears the software. As used herein, unless restricted to a non-transitory, tangible "storage" medium, a term such as computer- or machine"readable medium" refers to any medium that participates in providing instructions to a processor for execution.

Hence, a machine-readable medium, such as computerexecutable code, may take many forms including, but not limited to, a tangible storage medium, a carrier wave medium, or a physical transmission medium. A non-volatile storage medium includes, for example, an optical or a magnetic disk, such as any storage device of any computer, which may be used to implement the database and the like illustrated in the figure. A volatile storage medium includes a dynamic memory, such as a main memory of such a computer platform. A tangible transmission medium includes a coaxial cable, a copper wire, an optical fiber, and a wire that constitutes the bus within the computer system. A carrier-wave transmission medium may take the form of an electric or electromagnetic signal, or an acoustic wave or a light wave such as those generated during radio-frequency (RF) and infrared (IR) data communication. Thus, a typical form of the computer-readable medium includes, for example, a floppy disk, a flexible disk, a hard disk, a magnetic tape, and another magnetic medium; a CD-ROM, a DVD or DVD-ROM, and another optical medium; a punch card, a paper tape, and another physical storage medium with a hole pattern; a RAM, a ROM, a PROM, and an EPROM, a FLASH (registered trademark)-EPROM, and another memory chip or cartridge; a carrier wave carrying data or instructions, a cable or link carrying such carrier; or another medium from which a computer can read the programming code or information. Many of these forms of computer-readable media may be involved in carrying one or more sequences of one or more instructions to a processor for execution.

The computer system 101 can include or be in communication with, for example, the electronic display 125 including the user interface (UI) 140 for providing signals from the chip over time. Examples of the UI include, but are not limited to, a graphical user interface (GUI) and a webbased user interface.

The method and the system of the present disclosure can be implemented by one or more algorithms. The algorithms may be implemented by software when executed by the central processing unit 105.

The present disclosure provides software for causing a computer or the like to execute the method of the present disclosure, and a storage medium storing the software.

The present disclosure also provides the following embodiments:

A101 A method for evaluating a likelihood (certainty) that a subject belongs to a group for a classification attribute having a binary classification, the method comprising:

receiving a subject score for an observation value of the subject;
acquiring sensitivity and specificity of the subject score with the subject score as a parameter, by using a relational expression (already) established between the sensitivity and the specificity with a score for the observation value as a parameter;
acquiring a prior probability of an attribute of the subject; and
acquiring a likelihood of belonging to a classification attribute specific to the subject based on the sensitivity, the specificity, and the prior probability of the subject.

A102 The method according to embodiment A101, wherein
the acquiring of a likelihood of belonging to a classification attribute specific to the subject comprises acquiring a modified positive predictive value or a modified negative predictive value, respectively, for the subject score.
A201 A method for evaluating a likelihood (certainty) that a subject is positive or negative for a clinical examination having a binary classification, the method comprising:

receiving a subject score for a clinical examination of the subject;
acquiring sensitivity and specificity of the subject score with the subject score as a parameter, by using a relational expression established between the sensitivity and the specificity with a score for the clinical examination as a parameter;

acquiring prevalence of an attribute of the subject; and

acquiring a likelihood that the subject is positive or negative based on the sensitivity, the specificity, and the prevalence of the subject.

A202 The method according to embodiment A201, wherein

the acquiring of a likelihood that the subject is positive or negative comprises acquiring a modified positive predictive value or a modified negative predictive value, respectively, for the subject score.

A211 The method according to embodiment A201 or A202, wherein

the clinical examination is a biological examination.

A212 The method according to any one of embodiments A201 to A211, wherein

the clinical examination is a liquid biopsy.

A213 The method according to embodiment A212, wherein

the liquid biopsy is a urine examination or a blood examination.

A221 The method according to any one of embodiments A201 to A213, wherein

the clinical examination is a genetic examination.

A222 The method according to embodiment A221, wherein

the genetic examination is an RNA examination.

A223 The method according to embodiment A222, wherein

the genetic examination comprises examining a gene from urine.

A224 The method according to any one of embodiments A221 to A223, wherein

the genetic examination comprises examining a nucleic acid contained in an exosome.

A225 The method according to embodiment A224, wherein

the exosome is derived from urine.

A301 A method for evaluating a likelihood (certainty) that a subject belongs to a class for a classification attribute having an N- (N is a natural number) class classification, the method comprising:

receiving a subject score for an observation value of the subject;

acquiring, in an N-class classification obtained for a score for the observation value, a probability that a class i ($1 \le i \le N$, i is a natural number) is true (true "class i" rate) and a probability that the class i is false (false "class i" rate) of the subject score with the subject score as a parameter, by using a relational expression established between the true "class i" rate and the false "class i" rate, with the score as a parameter;

acquiring a prior probability of an attribute of the subject; and

acquiring a likelihood of belonging to the class i specific to the subject based on the true "class i" rate, the false "class i" rate, and the prior probability of the subject.

A302 The method according to embodiment A301, wherein

the acquiring of a likelihood of belonging to the class i specific to the subject based on the true "class i" rate, the false "class i" rate, and the prior probability of the subject comprises acquiring a conditional probability value of the subject score based on Bayesian statistics.

A303 The method according to embodiment A301, wherein

the acquiring of a likelihood of belonging to the class i specific to the subject based on the true "class i" rate, the false "class i" rate, and the prior probability of the subject comprises acquiring a true class i predictive value or a false class i predictive value for the subject score.

B101 A program for causing a computer to execute the method according to any one of embodiments A101 to A303.

C101 A computer-readable storage medium storing the program according to embodiment B101.

While preferred embodiments of the present disclosure have been illustrated and described herein, it will be obvious to those skilled in the art that such embodiments are provided by way of example only. It is not intended that the present disclosure be limited by the specific examples provided within the specification. While the present disclosure has been described with reference to the foregoing specification, the descriptions and illustrations of the embodiments herein are not meant to be construed in a limiting sense. Numerous modifications, changes, and substitutions will now occur to those skilled in the art without departing from the disclosure. Furthermore, it should be understood that all aspects of the present disclosure are not limited to the particular depictions, configurations, or relative proportions set forth herein, which depend upon a variety of conditions and variables. It should be understood that various alternatives to the embodiments of the disclosure described herein may be employed in practicing the invention. It is therefore contemplated that the present disclosure is intended to cover such alternatives, modifications, changes, or equivalents. It is intended that the following claims define the scope of the invention and that methods and structures within the claims and their equivalents be covered thereby.

Reference Signs List

| | |
|---|---|
| 100 | network system |
| 101 | computer system |
| 105 | central processing unit |
| 110 | memory |
| 115 | storage unit |
| 120 | communication interface |
| 125 | peripheral device |
| 130 | network |
| 140 | user interface |

**Claims**

1. A method for evaluating a likelihood that a subject belongs to a group for a classification attribute having a binary classification, the method comprising:

   receiving a subject score for an observation value of the subject;
   acquiring sensitivity and specificity of the subject score with the subject score as a parameter, by using a relational expression established between the sensitivity and the specificity with a score for the observation value as a parameter;
   acquiring a prior probability of an attribute of the subject; and
   acquiring a likelihood of belonging to a classification attribute specific to the subject based on the sensitivity, the specificity, and the prior probability of the subject.

2. The method according to claim 1, wherein
   the acquiring of a likelihood of belonging to a classification attribute specific to the subject based on the sensitivity comprises acquiring a modified positive predictive value or a modified negative predictive value, respectively, for the subject score.

3. A method for evaluating a likelihood that a subject is positive or negative for a clinical examination having a binary classification, the method comprising:

   receiving a subject score for a clinical examination of the subject;
   acquiring sensitivity and specificity of the subject score with the subject score as a parameter, by using a relational expression established between the sensitivity and the specificity with a score for the clinical examination as a parameter;
   acquiring prevalence of an attribute of the subject; and
   acquiring a likelihood that the subject is positive or negative based on the sensitivity, the specificity, and the prevalence of the subject.

4. The method according to claim 3, wherein
   the acquiring of a likelihood that the subject is positive or negative comprises acquiring a modified positive predictive value or a modified negative predictive value, respectively, for the subject score.

5. The method according to claim 3 or 4, wherein
   the clinical examination is a biological examination.

6. The method according to any one of claims 3 to 5, wherein
   the clinical examination is a liquid biopsy.

7. The method according to claim 6, wherein
   the liquid biopsy is a urine examination or a blood examination.

8. The method according to any one of claims 3 to 7, wherein
the clinical examination is a genetic examination.

9. The method according to claim 8, wherein
the genetic examination is an RNA examination.

10. The method according to claim 9, wherein
the genetic examination comprises examining a gene from urine.

11. The method according to any one of claims 8 to 10, wherein
the genetic examination comprises examining a nucleic acid contained in an exosome.

12. The method according to claim 11, wherein
the exosome is derived from urine.

13. A method for evaluating a likelihood that a subject belongs to a class for a classification attribute having an N-class classification, the method comprising:

receiving a subject score for an observation value of the subject;
acquiring, in an N-class classification obtained for a score for the observation value, a probability that a class i ($1 \leq i \leq N$) is true (true "class i" rate) and a probability that the class i is false (false "class i" rate) of the subject score with the subject score as a parameter, by using a relational expression established between the true "class i" rate and the false "class i" rate, with the score as a parameter;
acquiring a prior probability of an attribute of the subject; and
acquiring a likelihood of belonging to the class i specific to the subject based on the true "class i" rate, the false "class i" rate, and the prior probability of the subject.

14. The method according to claim 13, wherein
the acquiring of a likelihood of belonging to the class i specific to the subject based on the true "class i" rate, the false "class i" rate, and the prior probability of the subject comprises acquiring a conditional probability value of the subject score based on Bayesian statistics.

15. The method according to claim 13, wherein
the acquiring of a likelihood of belonging to the class i specific to the subject based on the true "class i" rate, the false "class i" rate, and the prior probability of the subject comprises acquiring a true class i predictive value or a false class i predictive value for the subject score.

16. A program for causing a computer to execute the method according to any one of claims 1 to 15.

START

S101

S102

S103

S104

END

FIG. 1

FIG. 2

EP 4 230 745 A1

## INTERNATIONAL SEARCH REPORT

| International application No. |
|---|
| **PCT/JP2021/037799** |

**A.    CLASSIFICATION OF SUBJECT MATTER**

*C12Q 1/68*(2018.01)i; *G16H 50/70*(2018.01)i; *G01N 33/493*(2006.01)i; *G01N 33/50*(2006.01)i; *G16Y 20/20*(2020.01)i; *G16Y 40/20*(2020.01)i

FI:    G16H50/70; G01N33/50 P; G01N33/493 A; G16Y20/20; G16Y40/20; C12Q1/68

According to International Patent Classification (IPC) or to both national classification and IPC

**B.    FIELDS SEARCHED**

Minimum documentation searched (classification system followed by classification symbols)

C12Q1/68; G16H50/70; G01N33/493; G01N33/50; G16Y20/20; G16Y40/20

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| X | MOSKOWITZ, C. S. PEPE, M. S. Quantifying and comparing the predictive accuracy of continuous prognostic factors for binary outcomes. Biostatistics. 01 January 2004, 5(1), pp. 113-127<br>   especially "3. Quantifying Predictive Accuracy and Differences in Predictive Accuracy" | 1-16 |
| P, X | FISCHER, F. Using Bayes theorem to estimate positive and negative predictive values for continuously and ordinally scaled diagnostic tests. International journal of methods in psychiatric research., 02 March 2021<br>"1 Introduction", "2 Methods" | 1-16 |
| A | JP 2007-529714 A (PERLEGEN SCIENCES, INC) 25 October 2007 (2007-10-25)<br>   abstract | 1-16 |

☐ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **05 November 2021** | **16 November 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

International application No.

**PCT/JP2021/037799**

| Patent document cited in search report | Publication date (day/month/year) | Patent family member(s) | Publication date (day/month/year) |
|---|---|---|---|
| JP 2007-529714 A | 25 October 2007 | US 2005/0196770 A1 abstract<br>WO 2005/086770 A2<br>CN 1950826 A | |

Form PCT/ISA/210 (patent family annex) (January 2015)